Europäisches Patentamt

European Patent Office

Office européen des brevets

(11) Publication number: **0 094 462**
**A1**

# EUROPEAN PATENT APPLICATION

(21) Application number: **82302622.4**

(22) Date of filing: **21.05.82**

(51) Int. Cl.³: **B 09 B 1/00**
**B 30 B 9/30, A 61 L 11/00**
**B 30 B 9/06**

(30) Priority: **13.05.82 GB 8213902**

(43) Date of publication of application:
**23.11.83 Bulletin 83/47**

(84) Designated Contracting States:
**CH DE FR IT LI NL SE**

(71) Applicant: **ESSO TEXTILE COMPANY LIMITED**
**No. 100-2 hsia kuai Rou Shan Road 1 Shan Li**
**Tanshui Taipei Hsien(TW)**

(72) Inventor: **Yen, Yi-Chen**
**No. 8-3 Alley 2 Lane 48, Sec 1 Chang Chiang Rd.**
**Pan-Chiao City Taipei Hsien(TW)**

(74) Representative: **Arthur, Bryan Edward**
**Withers & Rogers 4 Dyer's Buildings Holborn**
**London EC1N 2JT(GB)**

(54) **Waste matter processing.**

(57) Conventional methods of waste processing such as burying, incinerating or fermentating waste are typically costly, polluting and unhealthy. Waste matter may be processed by sterilising and compressing it into a solid block. The block may be stored in a hermetically sealed case (6). This method can be effectively performed safely and cleanly in a factory at low cost. The resulting garbage cases can be used in road foundations, making embankments and slopes, filling holes in the ground or in building dams.

EP 0 094 462 A1

./...

Croydon Printing Company Ltd.

Fig.1

-1-

Waste Matter Processing.

This invention relates to waste matter processing, that is the disposal of domestic and commercial refuse. Waste should be hygenically disposed of with minimal pollution. The rapid development of towns and cities world-wide and the overall increase in population has increased the importance of finding an effective way of disposing of waste.

In one conventional method of waste disposal, the waste is buried but this takes up large areas of land. Burying sites are generally out of urban areas and so much fuel and time is used in transporting the waste thereto. Such sites could be extremely unhealthy and lead to various types of pollution e.g. water pollution.

Incinerating waste is an alternative conventional method but this has several drawbacks. The useful life of an incinerator is limited; the incinerator will be expensive to buy, run and maintain. Non-flammable refuse should not be burnt and must be separated from the flammable refuse. The smoke produced by the incineration may be poisonous and it may pollute the atmosphere.

Waste can thirdly be fermented and used as fertiliser. Again, large areas of land are needed for this. This land must be located well-away from populated areas to avoid posing a health risk. Furthermore, this is a costly method as special chemicals are needed. The fermentation also takes 3 or 4 weeks to fully take place. Both this method and that of burying are also environmentally unattractive.

A way of waste disposal is therefore required in which the above problems are minimised.

According to this invention, we propose a method of waste processing, comprising sterilising a quantity of waste matter, compressing the sterilised waste matter and hermetically sealing the compressed waste matter in a container.

This method has several advantages in that it can be effectively performed safely and cleanly in a factory at low cost. The method can be done automatically, thus saving manpower.

The container can be easily stored and transported. It can be used usefully, for example in building dams, filling holes in the ground, in making embankments and slopes, and in road foundations.

An example of the invention is described with reference to the drawings, in which:

Figure 1 is a flow chart showing a method of waste processing according to this invention;

Figure 2 shows a side view of a conveyor and mould used in the invention;

Figure 3 shows a plan view of the mould;

Figure 4 shows a more detailed view of the mould;

Figure 5 shows four compressors mounted on one rig;

Figure 6 shows the four compressors each in the mould;

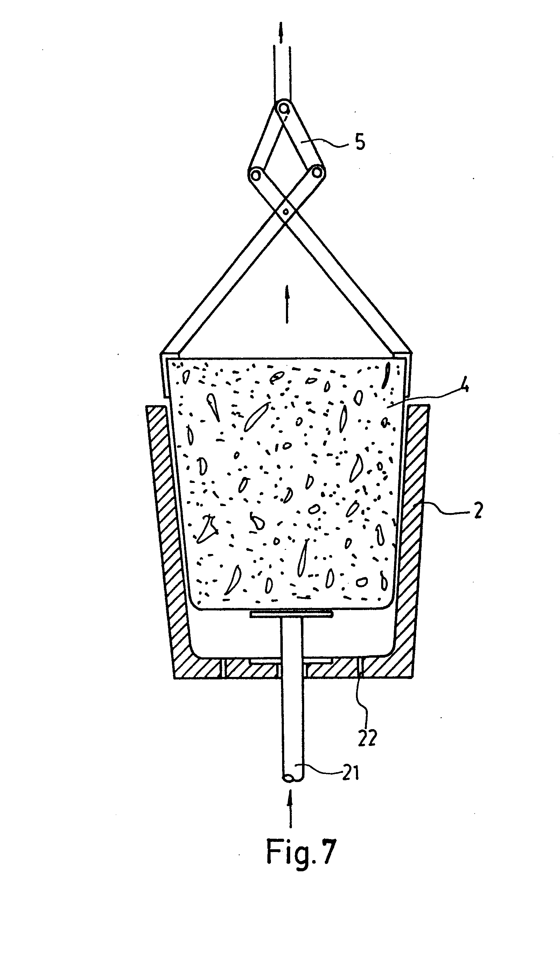

Figure 7 shows a compressed garbage block being lifted out of the mould; and

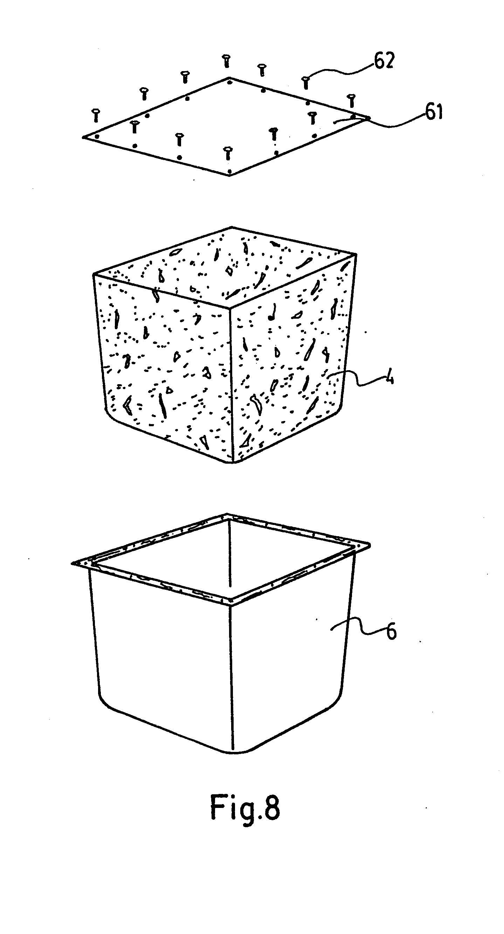

Figure 8 is an exploded view showing the compressed garbage block and a casing in which the compressed garbage block is sealed.

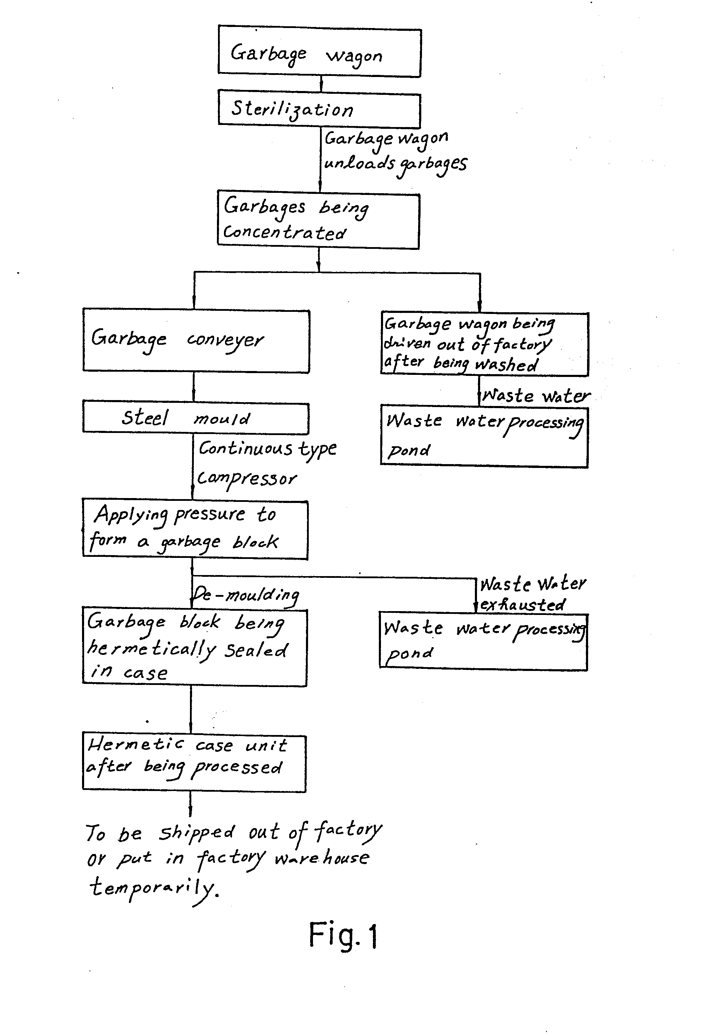

The steps of the method of waste processing, as shown in Figure 1, are as follows. First of all, the garbage usually in a wagon is sterilised. Then, the wagon is unloaded and sterilised again.

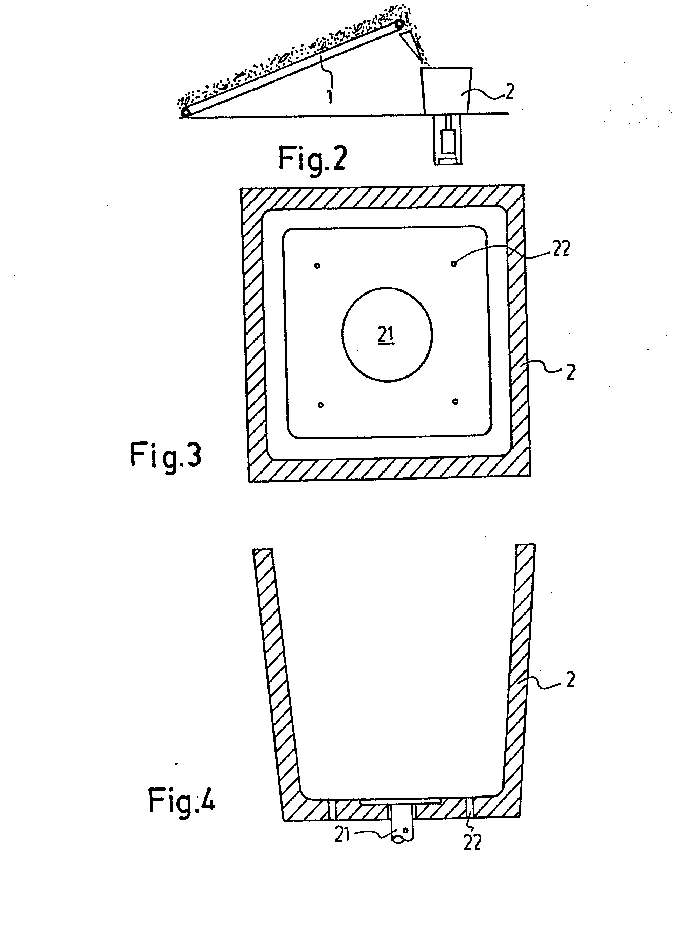

The garbage is fed on a converyor (1), as shown in Figure 2, into a steel mould (2). The base of the mould has holes (22) through which liquid may escape. Also, hydraulic lifting means (2) may be operated through the base.

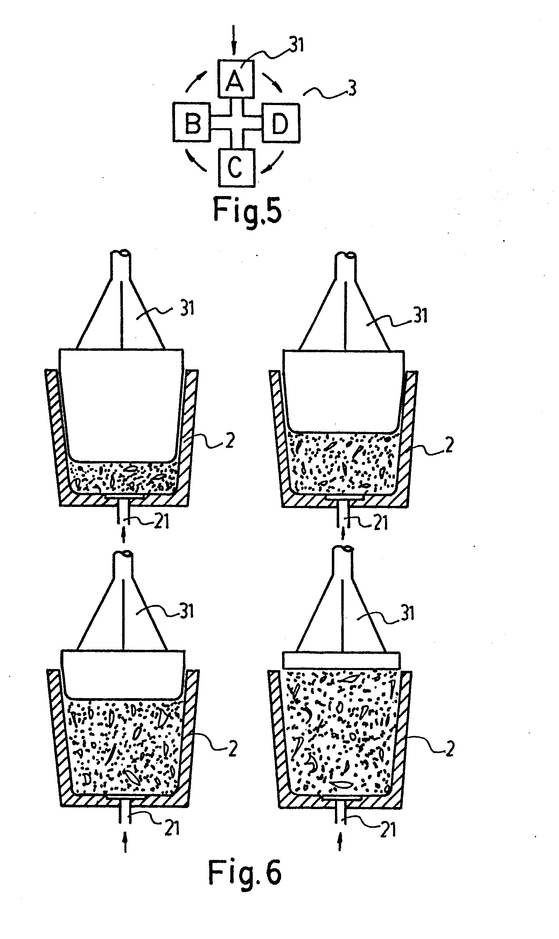

A compressor is then placed on the top of the garbage and started. Four decreasing sizes of compressor A, B, C, D, may be used consecutively, as shown in Figure 6 and more garbage can be added after each compressor has acted on the garbage.

-4-

The compressors may be automatically moved into and out of position by one rig (31) on which they are mounted. During the compression, water in the garbage is forced out of the mould via the holes (22).

When the mould is full of fully compressed garbage, the solid garbage block may be removed by the lifting means (2) pushing upwards on the base of the garbage block and a clamp (5), clamped to the top of the garbage, pulling the garbage block upwards.

The block is fitted into a F.R.P. case and taken for sealing. The case is coated along the open edge with a resin. The lid is placed over the opening and locked in place e.g. by screws so that the unit is hermetically sealed. This unit is then ready for use.

Claims

1.    A method of waste processing, comprising sterilising a quantity of waste matter, compressing the sterilised waste matter and hermetically sealing the compressed waste matter in a container.

2.    A method of waste processing, according to claim 1, wherein the waste matter is compressed in a mould of the same internal shape and size as the hermetically sealed container and the sterilised waste matter is fully compressed into a solid block.

3.    A method of waste processing, according to claim 1 or claim 2, wherein four compressors of different, decreasing sizes are used consecutively and more garbage is added between compressors.

4.    Apparatus for waste processing including a compressing mould having holes through which liquid may escape.

5.    Apparatus for waste processing according to claim 4, wherein lifting means is movable through the base of the mould.

6.    Apparatus for waste processing according to claim 4, wherein the waste is supplied to the mould on a conveyor.

7.    Apparatus for waste processing according to claim 4, including four compressors A, B, C, and D of different decreasing sizes mounted on one rig for use consecutively.

```
        ┌──────────────────┐
        │  Garbage wagon   │
        └────────┬─────────┘
                 │
        ┌────────┴─────────┐
        │  Sterilization   │
        └────────┬─────────┘
                 │  Garbage wagon
                 │  unloads garbages
        ┌────────┴─────────┐
        │  Garbages being  │
        │  Concentrated    │
        └────────┬─────────┘
                 │
      ┌──────────┴───────────────────────┐
      │                                  │
┌─────┴──────────┐          ┌────────────┴─────────┐
│  Garbage       │          │ Garbage wagon being  │
│  conveyer      │          │ driven out of factory│
│                │          │ after being washed   │
└─────┬──────────┘          └────────────┬─────────┘
      │                                  │ Waste water
┌─────┴──────────┐          ┌────────────┴─────────┐
│  Steel  mould  │          │ Waste water processing│
└─────┬──────────┘          │ pond                  │
      │ Continuous type     └──────────────────────┘
      │ Compressor
┌─────┴──────────┐
│ Applying pressure to│
│ form a garbage block│
└─────┬──────────┘
      │                              Waste Water
      │                              exhausted
      │ De-moulding    ┌────────────────────────┐
┌─────┴──────────┐     │ Waste water processing │
│ Garbage block being │     │ pond                   │
│ hermetically Sealed │     └────────────────────────┘
│ in case             │
└─────┬──────────┘
      │
┌─────┴──────────┐
│ Hermetic case unit │
│ after being processed│
└─────┬──────────┘
      │
```

To be shipped out of factory
or put in factory warehouse
temporarily.

Fig. 1

Fig.2

Fig.3

Fig.4

Fig.5

Fig.6

4/5

Fig. 7

62

61

4

6

Fig.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | FR-A-2 078 203 (SEADUN) * page 1, lines 19-29; page 2, line 14 to page 5, line 21; figures 1-3 * | 1,6 | B 09 B 1/00 B 30 B 9/30 A 61 L 11/00 B 30 B 9/06 |
| X | FR-A-2 073 947 (SETIR) * page 7, line 16 to page 10, line 31 ; figures 2-5 * | 4,6 | |
| X | FR-A-1 600 245 (RIES) *Page 2, lines 10-42; figures 1,2* | 4 | |
| A | US-A-3 547 577 (C.L.LOVERCHECK) * the whole document * | 1 | |
| A | US-A-2 984 957 (G.LUNDGREN) * the whole document * | 3 | **TECHNICAL FIELDS SEARCHED (Int. Cl. ³)** |
| A | GB-A-1 151 584 (BELOIT CO.) * page 2, line 30 - page 3, line 25; figures 1-3 * | 5 | B 09 B A 61 L B 30 B |
| A | DE-A-2 407 487 (MIRGEL GOTTFRIED) | | |
| A | FR-A-2 047 367 (INTERNATIONAL DYNETICS CORP.) | | |
| | --- -/- | | |

The present search report has been drawn up for all claims

| Place of search | Date of completion of the search | Examiner |
|---|---|---|
| THE HAGUE | 05-08-1982 | LAVAL J.C.A |

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl. ³) |
| A | GB-A- 132 958 (HYDRAULIC ENGINEERING COMPANY LTD.) | | |

-----

TECHNICAL FIELDS
SEARCHED (Int. Cl. ³)

The present search report has been drawn up for all claims

| Place of search THE HAGUE | Date of completion of the search 05-08-1982 | Examiner LAVAL J.C.A |
|---|---|---|